# EUROPEAN PATENT SPECIFICATION

(11) **EP 2 792 361 B1**
(45) Date of publication and mention of the grant of the patent: **27.03.2019**
(21) Application number: 12856624.7
(22) Date of filing: 17.12.2012
(51) Int. Cl.: A61K 31/7032, A61P 25/00, A61P 25/16, A61P 25/28, A61K 36/64

(54) **MEDICAL COMPOSITION FOR PREVENTING OR TREATING AMYLOID BETA PEPTIDE RELATED DISEASES OR CONDITIONS**
MEDIZINISCHE ZUSAMMENSETZUNG ZUR PRÄVENTION ODER BEHANDLUNG VON AMYLOID-BETA-PEPTID-VERMITTELTEN ERKRANKUNGEN ODER LEIDEN
COMPOSITION MÉDICALE POUR PRÉVENIR OU TRAITER DES ÉTATS OU MALADIES ASSOCIÉS AU PEPTIDE BÉTA AMYLOÏDE

(30) Priority: 16.12.2011 US 201161576367 P
(43) Date of publication of application: 22.10.2014
(73) Proprietor: Sinphar Tian-li Pharmaceutical Co., Ltd. (Hangzhou), Yuhang Economic Development Zone Hangzhou Zhejiang 311100 (CN)
(72) Inventor: LIN, Hang-Ching, Taipei City Taiwan (TW); SU, Muh-Hwan, I Lan Taiwan (TW); HUANG, Young-Ming, I Lan Taiwan (TW); TANG, Jing-Jing, I Lan Taiwan (TW)
(74) Representative: Grünecker Patent- und Rechtsanwälte PartG mbB
(86) International application number: PCT/CN2012/086796
(87) International publication number: WO 2013/087042

(56) References cited:
- EP-A1- 2 397 144
- CN-A- 1 526 400
- JP-A- 2009 196 905
- US-A1- 2004 162 246
- KOO KYUNG AH ET AL: "In vitro neuroprotective activities of phenylethanoid glycosides from Callicarpa dichotoma", PLANTA MEDICA, vol. 71, no. 8, August 2005 (2005-08), pages 778-780, XP055180316, ISSN: 0032-0943
- WANG H ET AL: "Acteoside protects human neuroblastoma SH-SY5Y cells against beta-amyloid-induced cell injury", BRAIN RESEARCH, ELSEVIER, AMSTERDAM, NL, vol. 1283, 4 August 2009 (2009-08-04), pages 139-147, XP026350245, ISSN: 0006-8993 [retrieved on 2009-06-09]
- DATABASE EMBASE [Online] ELSEVIER SCIENCE PUBLISHERS, AMSTERDAM, NL; January 2007 (2007-01), ZHAO L ET AL: "Neuroprotective effect of acteoside against MPTP-induced mouse model of Parkinsons disease", XP002737966, Database accession no. EMB-2007207436 & CHINESE PHARMACOLOGICAL BULLETIN 200701 CN, vol. 23, no. 1, January 2007 (2007-01), pages 42-46, ISSN: 1001-1978
- LU CHUN-HUA ET AL.: 'Water-soluble Constituents from Callicarpa pedunculata.' CHINESE JOURNAL OF NATURAL MEDICINES. vol. 6, no. 3, May 2008, pages 176 - 178, XP008171345

## Description

### Technical Field

The present invention relates to a pharmaceutical composition for use in a method of preventing or treating certain amyloid β peptide associated diseases or conditions. The pharmaceutical composition comprises acteoside and isoacteoside as potent components capable of inhibiting formation, accumulation or aggregation of amyloid beta peptides.

### Background Techniques

US patent No. 7,087,252 B2 discloses a medicinal preparation containing phenylethanoid glycosides extracted from *Cistanche tubulosa* (Schenk.) Wight, said preparation comprising 25-50 wt% of echinacoside and 5-15 wt% of acteoside, which is useful in treating senile dementia. Isoacteoside and other phenylethanoid glycosides are known also being contained in said medicinal preparation.

Chun-Hua and Yue-Mao (2008), Chin J. Nat. Med. Vol. 6, No. 3, pg. 176-178, describes watersoluble constituents from Callicarpa pedunculata. CN1526400 (A) describes a tubiflorous desert cistanche preparation containing phenethyl alcohol glycoside and its preparation process and use. Koo et al (2005), Planta Med., 71, pg. 778-780, describes in vitro neuroprotective activities of phenylethanoid glycosides from Callicorpa dichotoma. JP2009196905 describes a liver protective agent or anti-TNF alpha agent obtained from Cistanche tubulosa (Shrenk) R. Wright. Wang et al. (2009), Brain Research, 1283, pg. 139-147, describes that acteoside protects human neuroblastoma SH-SYSY cells against beta-amyloid-induced cell injury. Zhao et al. (2007), Chinese Pharmacological Bulletin, 23, pg. 42-46, describes the neuroprotective effect of acteoside against an MPTP-induced mouse model of Parkinsons disease.

The applicant of this application in WO 2011/157059 A1 discloses use of isoacteoside or a pharmaceutically acceptable salt thereof in inhibiting the formation, accumulation or aggregation of amyloid β peptide (Aβ), and use in the fabrication of a medicament for preventing or treating Aβ-associated diseases or conditions.

In the present application, the inventors continue the research of WO 2011/157059 A1 and obtain a related inventive accomplishment.

### Summary of the Invention

The subject matter of the invention is as set out in the appended claims.

Since Aβ and its aggregates are likely to cause various diseases or conditions in organisms, one object of the present invention is to provide pharmaceutical composition for inhibiting formation, accumulation or aggregation of Aβ, and such pharmaceutical composition can be used as an additive in food, drinks, chewing substance, patches, skin care products, etc.. Another object of the present invention is to provide a pharmaceutical composition for use in a method of preventing or treating Aβ-associated diseases or conditions.

Still another object of the present application is to provide use of a pharmaceutical composition in the fabrication of a medicament for preventing or treating Aβ-associated diseases or conditions.

A pharmaceutical composition for use in a method of preventing or treating Aβ-associated diseases or conditions provided in accordance with the present invention comprises acteoside and isoacteoside as potent components, wherein a weight ratio of the isoacteoside to the acteoside is 4:1 to 2:3.

Preferably, the pharmaceutical composition for use according to the invention is free of echinacoside.

Preferably, the pharmaceutical composition for use according to the invention is able to inhibit formation, accumulation or aggregation of amyloid β peptides.

Preferably, the pharmaceutical composition for use according to the invention is able to inhibit extracellular formation, accumulation or aggregation of amyloid β peptides.

Preferably, the pharmaceutical composition for use according to the invention is able to inhibit neuronal damage or apoptosis caused by the amyloid β peptides, so as to retain, improve or restore learning and memory abilities.

The Aβ-associated disease or condition treated or prevented by the pharmaceutical composition for use according to the invention is Alzheimer's disease, mild cognitive impairment, Lewy body dementia, Down syndrome, Hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, or Pick's disease.

Preferably, the pharmaceutical composition for use according to the invention is for treating Alzheimer's disease.

Preferably, the pharmaceutical composition for use according to the invention is for preventing an organism from suffering Alzheimer's disease or for delaying an organism suffering Alzheimer's disease.

Preferably, an effective dosage of the pharmaceutical composition for use according to the invention to a person is equivalent to 0.2 mg to 4.0 mg of the potent components per kg of body weight per day.

Preferably, the pharmaceutical composition for use according to the invention comprises a phenylethanoid glycoside preparation extracting from a plant as a source of the potent components, wherein the preparation comprises the isoacteoside to the acteoside as the major phenylethanoid glycosides, and the content of the isoacteoside is greater than that of the acteoside.

Preferably, the preparation comprises 12-32% of acteoside and 26-46% of the isoacteoside, based on the weight of the preparation.

Preferably, the plant is *Cistanche tubulosa* (Schenk.) Wight.

Preferably, the preparation is provided by a process comprising the following steps:
a) extracting fleshy stems of *Cistanche tubulosa* (Schenk.) Wight with a first polar solvent;
b) introducing the resulting extract from step a) into a column which is packed with hydrophobic macro-porous polymeric beads, thereby enabling phenylethanoid glycosides to be adsorbed on the polymeric beads;
c) eluting the column by use of a second polar solvent serving as a mobile phase, so that relatively less strongly adsorbed compounds are eluted from the column with most of phenylethanoid glycosides still being adsorbed on the polymeric beads; and
d) eluting the column by use of a third polar solvent so as to obtain an eluate which contains phenylethanoid glycosides, wherein the first polar solvent is water, methanol, ethanol, a mixture of water and methanol, or a mixture of water and ethanol; the second polar solvent is water; and the third polar solvent is methanol, ethanol, a mixture of water and methanol, or a mixture of water and ethanol, and the third polar solvent is lower in polarity than the second polar solvent;
e) concentrating the eluate which contains phenylethanoid glycosides, dissolving the concentrate in water, and contacting the aqueous solution with a macro-porous resin, so that the phenylethanoid glycosides are adsorbed on the macro-porous resin; and
f) eluting the macro-porous resin with a fourth polar solvent and a fifth polar solvent in sequence, wherein the fifth polar solvent is lower in polarity than the fourth polar solvent, so that an eluate resulting from the fourth polar solvent elution does not contain acteoside and isoacteoside, and an eluate resulting from the fifth polar solvent elution contain only acteoside and isoacteoside, wherein the fourth polar solvent and the fifth polar solvent are a mixture of water and methanol or a mixture of water and ethanol.

Preferably, the fourth polar solvent is 25-35% ethanol aqueous solution and the fifth polar solvent is 35-45% ethanol aqueous solution.

To better understand the above and other objects, features and advantages of the present invention, the present invention will be described in detail below with examples presented with reference to the annexed drawings.

### Brief Description of the Drawings

Fig. 1 shows the effects of drug A (acteoside), drug I (isoacteoside), C (control group, no drug), and pharmaceutical compositions having different ratios of A to I on extracellular Aβ1-40 accumulation.
Fig. 2 shows the effects of drug A (acteoside), drug I (isoacteoside), C (control group, no drug), and pharmaceutical compositions having different ratios of A to I on Aβ1-42 on Aβ1-42 oligomerization.

### Best Modes of Embodying the Invention

Various diseases caused by Aβ have a common feature: formation of Aβ aggregates. These Aβ aggregates present in shapes such as fibrils or plaques, and deposit in systems, organs, tissues or body fluids of organisms, causing various diseases or conditions. It is therefore supposed that inhibition of Aβ formation, accumulation or aggregation can be used as an approach for effectively preventing or treating Aβ-associated diseases or conditions.

The term "prevent" used herein means avoiding or delaying occurrence of a disease or condition in organisms. The term "treat" used herein means slowing or stopping progress of a disease or condition, or making an individual return back to his improved or normal status.

The term "amyloid β peptide (Aβ)-associated diseases or conditions" generally refers to those diseases or conditions that occur relating to formation, accumulation or aggregation of Aβ, and particularly refers to the diseases or conditions that are caused by Aβ. When abnormal formation, accumulation or aggregation is found in a certain proportion of individuals with certain diseases or conditions, the diseases or conditions can be considered as being associated with Aβ. In addition, when Aβ aggregates somewhere that is close to occurrence of pathological features affected in certain diseases or conditions, the diseases or conditions can be also considered as being associated with Aβ.

In the following examples test samples listed in Table 1 were used for carrying out the Aβ experiments, which were compared to a Vehicle control group which was not added with any test samples.

**Table 1: Test samples**

| Symbol | Test sample | Concentration | Source |
|---|---|---|---|
| A | Acteoside | 50 µg/ml | Sinphar Lab., purity 97% |
| I | Isoacteoside | 50 µg/ml | Sinphar Lab. , purity 97% |
| A:I 40:10 | Acteoside + Isoacteoside | 40 µg/ml + 10 µg/ml | Sinphar Lab. , purity 97% |
| A:I 30:20 | Acteoside + Isoacteoside | 30 µg/ml + 20 µg/ml | Sinphar Lab. , purity 97% |
| A:I 20:30 | Acteoside + Isoacteoside | 20 µg/ml + 30 µg/ml | Sinphar Lab. , purity 97% |
| A:I 10:40 | Acteoside + Isoacteoside | 10 µg/ml + 40 µg/ml | Sinphar Lab. , purity 97% |

### Example 1: Neuroblastoma cell culture

Wild-type human neuroblastoma cells (SH-SY5Y) were cultured in Eagle's Minimum essential Medium (EMEM) / Ham's F12 medium (1:1 mixture) (containing 10% FBS, 10 units/ml penicillin, 10µg/ml Streptomycin). Wild-type mouse neuroblastoma Neuro-2a cells were cultured in minimum essential medium (MEM) (containing 10% FBS, 10 units/ml penicillin, 10µg/ml Streptomycin).

### Example 2: The effect of each test sample on extracellular Aβ1-40 accumulation

The medium of the wild-type human neuroblastoma SH-SY5Y cells in Example 1 were switched into chemical defined medium (EMEM/F12 medium (Cat.No.12500-062), Hepes 5 mM, Glucose 0.6%, NaHCO₃ 3 mM, Glutamine 2.5 mM, Insulin 25 µg/ml, Transferin 100 µg/ml, Progestrone 20 nM, Putrescine 60 µM, Sodium selenite 30 nM, Heparin 2 µg/ml). Each well contained 1x10⁵ SH-SY5Y cells in 300 µl of culture medium. Thirty minutes later, each well was treated with the test samples given in Table 1 respectively at a total concentration of 50 µg/ml for 24 hours. After that, the level of Aβ1-40 in the medium of each well was analyzed by Human Aβ1-40 immunoassay kits (Catalog # KHB3482 Invitrogen).

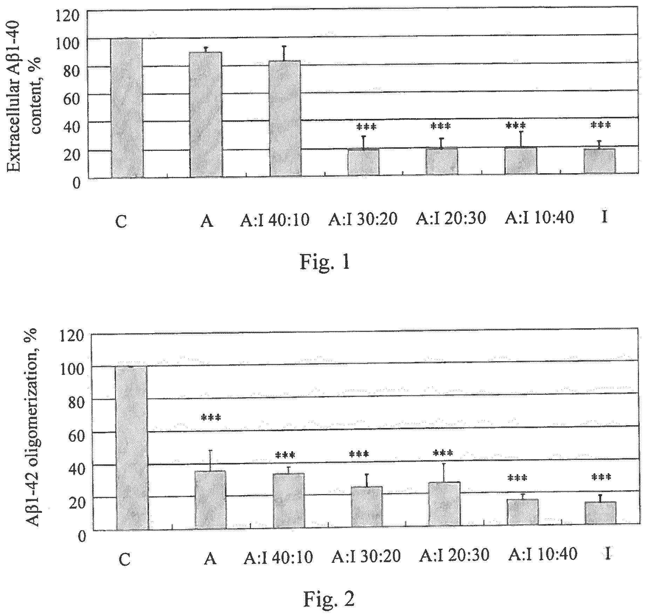

Human neuroblastoma SH-SY5Y cells cause extracellular accumulation of Aβ. Fig. 1 shows the percentage of Aβ1-40 in the medium of each SH-SY5Y well treated with the test samples, based on the amount of Aβ1-40 in the medium of the Vehicle control group (C) which was not treated with any test sample. The results were shown in mean ± standard deviation (SD) form. Significant difference between the Vehicle control group and the test sample-treated groups were indicated by ***, P<0.001.

Referring to Fig. 1, the test sample A (acteoside) reduces the level of Aβ1-40 in the medium by about 10%, the test sample A:I 40:10 (acteoside 40 µg/ml + isoacteoside 10 µg/ml) reduces the level of Aβ1-40 in the medium by about 22%, the remaining test samples reduce the level of Aβ1-40 in the medium by about 80%. The results in Fig. 1 indicate that the test samples of acteoside + isoacteoside = 30 µg/ml + 20 µg/ml; 20 µg/ml + 30 µg/ml; and 10 µg/ml + 40 µg/ml, and the test sample of isoacteoside = 50 µg/ml possess significant activity on reducing extracellular Aβ1-40 accumulation.

### Example 3: The effect of each test samples on Aβ1-42 oligomerization

Dried Human Aβ1-42 was taken out from the refrigerator and equilibrated to room temperature. Aβ1-42 was dissolved in 1,1,1,3,3,3-Hexa-fluro-2-propanol (HFIP) to a concentration of 1mM, and was then placed at room temperature for one hour. The Aβ1-42/HFIP solution was aliquoted by Hamilton syringe, and was then dried under a stream of nitrogen gas, followed by storing at a temperature of -20°C. Aβ1-42 treated with HFIP was dissolved in PBS, and was vibration-incubated with treatment of each test sample at a concentration of 50 µg/ml and at 4°C for 24 hours to prepare Aβ1-42 oligomers. The level of Aβ1-42 oligomerization was analyzed by thioflavin T fluorescence (Ex=450 nm, Em=482 nm).

Fig. 2 shows the effects of the test samples in Table 1 on Aβ1-40 oligomerization, and the results are shown in percentage based on the control group (C) which was not treated with any test sample. The results in Fig. 2 indicate that all the test samples were found to possess activities on inhibiting Aβ1-42 oligomerization, wherein the test samples of acteoside + isoacteoside = 30 µg/ml + 20 µg/ml; 20 µg/ml + 30 µg/ml; and 10 µg/ml + 40 µg/ml, and the test sample of isoacteoside = 50 µg/ml possess better activity on inhibiting Aβ1-42 oligomerization. That is to say, these pharmaceutical compositions can be used to prevent or treat Aβ-associated diseases or conditions.

The described Aβ-associated diseases or conditions to be treated or prevented in the method in which the inventive PC is to be used are selected from the group consisting of Alzheimer's disease, mild cognitive impairment, Lewy body dementia, Down syndrome, hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, and Pick's disease. In addition, even though the described Aβ is exemplified by Aβ1-40 at most or highly fibrillogenic Aβ1-42, the Aβ can also comprise other peptide fragments.

The results in Figs. 1 and 2 show that isoacteoside possesses a better activity; however, in realizing the application of isoacteoside which is a saccharide-containing molecule is difficult to be chemically synthesized. It is also very costive for obtaining a high purity isoacteoside from the source of a plant. Taking the practical application aspects into consideration such as the economic benefit and the medical treatment effectiveness, the results in Figs. 1 and 2 indicate that the mixtures of acteoside and isoacteoside possessing an activity comparable to the pure isoacteoside as an amyloid β peptide inhibitor can be used as an alternative of the purified isoacteoside in preventing or treating amyloid β peptide-associated diseases or conditions.

In the following example, the process for preparing a phenylethanoid glycoside-containing preparation disclosed in US patent No. 7,087,252 was adopted, which comprises the following steps: a) extracting subterranean portions of (sufleshy stems) of *Cistanche tubulosa* (Schenk.) Wight with a first polar solvent; b) introducing the resulting extract from step a) into a column which is packed with hydrophobic macro-porous polymeric beads, thereby enabling phenylethanoid glycosides to be adsorbed on the polymeric beads; c) eluting the column by use of a second polar solvent serving as a mobile phase, so that relatively less strongly adsorbed compounds are eluted from the column with most of phenylethanoid glycosides still being adsorbed on the polymeric beads; and d) eluting the column by use of a third polar solvent so as to obtain an eluate which contains phenylethanoid glycosides, wherein the third polar solvent is lower in polarity than the second polar solvent.

The first polar solvent in step a) can be for example water or a mixed solvent of water and ethanol. The second polar solvent in step c) is water. The third polar solvent in step d) can be for example methanol, ethanol, a mixed solvent of water and methanol, or a mixed solvent of water and ethanol, wherein the third polar solvent is a mixed solvent of water and ethanol.

The present invention provides a further purification process to obtain a pharmaceutical composition for use according to the invention an comprising acteoside and isoacteoside which are the only phenylethanoid glycosides contained therein by directly purifying the aforesaid phenylethanoid glycosides-containing preparation from *Cistanche tubulosa* (Schenk.) Wight. The further purification process comprises the steps of: e) purifying the aforesaid preparation from *Cistanche tubulosa* (Schenk.) Wight containing various phenylethanoid glycosides with a macro-porous resin; and f) eluting the macro-porous resin with a fourth polar solvent and a fifth polar solvent in sequence, wherein the fifth polar solvent is lower in polarity than the fourth polar solvent, so that an eluate resulting from the fifth polar solvent elution contains substantially only acteoside and isoacteoside of the phenylethanoid glycosides. In one of the preferred embodiments of the present invention the fourth polar solvent can be for example 25-35% ethanol aqueous solution and the fifth polar solvent can be for example 35-45% ethanol aqueous solution.

Preferably, the hydrophobic macro-porous polymeric beads are cross-linked polyaromatics, and more preferably cross-linked polystyrene or cross-linked copolymer of styrene and divinyl benzene, such as D-101 type or AB-8 type materials.

A pharmaceutical composition for use according to the invention contains substantially only acteoside and isoacteoside of the phenylethanoid glycosides can be directly obtained by concentrating or drying the eluate resulting from the fifth polar solvent elution.

### Example 4: A pharmaceutical composition contains substantially only acteoside and isoacteoside of the phenylethanoid glycosides

10 kg of the flakes of fleshy stems of *Cistanche tubulosa (Schenk.) Wight* was soaked in water in an amount which was 8 times of the flakes. The flakes was soaked in the water for one hour before being decocted with the water for two hours. The decocted mixture was filtered to obtain a first filtrate. The residue was then decocted with the water in an amount which was 6 times of the residue and the decocted mixture was filtered to obtain a second filtrate. A third filtrate was also obtained by the same procedures as the second filtrate. The three filtrates were combined and concentrated in vacuo to have a specific gravity of 1.10 (50°C). The filtrate in the concentrated form was mixed with ethanol to form a mixture containing 60% of the ethanol, which was then refrigerated for 12 hours. Thereafter, a supernatant was harvested from the cooled mixture while the residue was filtered to obtain a filtrate, which was combined with the supernatant followed by concentrating in vacuo to obtain an end extract having a specific gravity of 1.10 (50°C).

6 kg of the end extract was dissolved in water with heating, which was in the same amount of the end extract. The extract solution was then applied into an adsorption column packed with macro-porous adsorption resin. The column was first eluted with water to yield a water eluate in the amount of four times of the fleshy stems, and was than eluted with 40% ethanol to yield a first 40% ethanol eluate in the amount of five times of the fleshy stems. The water eluate was subjected to another round of the adsorption-desorption operations by eluting the column with water in the amount of three times of the fleshy stems and with 40% ethanol in sequence to obtain a second ethanol eluate in the amount of four times of the fleshy stems. The two 40% ethanol eluates were combined, concentrated, and dried to yield a preparation containing phenylethanoid glycosides and having a weight of 1107 g.

A high performance liquid chromatography (HPLC) was carried out under the following conditions: solvent A: acetonitrile containing 0.1% formic acid (CAN); solvent B: MQ-H₂O containing 0.1% formic acid; column: Agilent Zorbax SB-C18 column of 2.1 x 150 mm, 5 µm; flow rate: 0.3 ml/min; and UV wavelength of 333 nm. The contents of echinacoside, acteoside and isoacteoside of the preparation containing phenylethanoid glycosides were measured, which were calculated as 33.6 wt%, 3.65 wt% and 6.05 wt%, respectively.

200 g of the preparation containing phenylethanoid glycosides was dissolved in 800 g of water, and the resulting solution was introduced into a macro-porous resin to undergo purification, which was eluted with 30% ethanol aqueous solution and 40% ethanol aqueous solution in sequence. A thin layer chromatography was conducted with UV 365 nm to analyze each eluate, wherein the eluate collected from the 30% ethanol aqueous solution does not contain acteoside and isoacteoside, and the eluate collected from the 40% ethanol aqueous solution contain only acteoside and isoacteoside of phenylethanoid glycosides of 23.6 g. In this example, the acteoside in the eluate is 22.5 wt%, and the isoacteoside in the eluate is 36.4 wt%.

## Claims

1. A pharmaceutical composition for use in a method of preventing or treating amyloid β peptide-associated diseases or conditions in an organism, said composition comprising acteoside and isoacteoside as potent components, wherein a weight ratio of the isoacteoside to the acteoside is 4:1 to 2:3, wherein the amyloid β peptide-associated disease or condition is Alzheimer's disease, mild cognitive impairment, Lewy body dementia, Down syndrome, Hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, or Pick's disease.

2. The pharmaceutical composition for use as in claim 1, which is free of echinacoside.

3. The pharmaceutical composition for use as in claim 1, wherein the pharmaceutical composition inhibits formation, accumulation or aggregation of the amyloid β peptides.

4. The pharmaceutical composition for use as in claim 1, wherein said pharmaceutical composition is for treating Alzheimer's disease.

5. The pharmaceutical composition for use as in claim 1, wherein said pharmaceutical composition is for preventing an organism from suffering Alzheimer's disease or for delaying an organism suffering Alzheimer's disease.

6. The pharmaceutical composition for use as in claim 1, wherein an affective dosage of said pharmaceutical composition to human is equivalent to 0.2 mg - 4.0 mg of said potent components per kg of body weight per day.

7. The pharmaceutical composition for use as in claim 2, which comprises a phenylethanoid glycoside preparation extracting from a plant as a source of the potent components, wherein the preparation comprises the isoacteoside and the acteoside as the major phenylethanoid glycosides, and the content of the isoacteoside is greater than that of the acteoside.

8. The pharmaceutical composition for use as in claim 7, wherein the preparation comprises 12-32% of acteoside and 26-46% of the isoacteoside, based on the weight of the preparation.

9. The pharmaceutical composition for use as in claim 8, wherein the plant is *Cistanche tubulosa* (Schenk.) Wight.

10. The pharmaceutical composition for use as in claim 9, wherein the preparation is provided by a process comprising the following steps:
a) extracting fleshy stems of *Cistanche tubulosa* (Schenk.) Wight with a first polar solvent;
b) introducing the resulting extract from step a) into a column which is packed with hydrophobic macro-porous polymeric beads, thereby enabling phenylethanoid glycosides to be adsorbed on the polymeric beads;
c) eluting the column by use of a second polar solvent serving as a mobile phase, so that relatively less strongly adsorbed compounds are eluted from the column with most of phenylethanoid glycosides still being adsorbed on the polymeric beads; and
d) eluting the column by use of a third polar solvent so as to obtain an eluate which contains phenylethanoid glycosides, wherein the first polar solvent is water, methanol, ethanol, a mixture of water and methanol, or a mixture of water and ethanol; the second polar solvent is water; and the third polar solvent is methanol, ethanol, a mixture of water and methanol, or a mixture of water and ethanol, and the third polar solvent is lower in polarity than the second polar solvent;
e) concentrating the eluate which contains phenylethanoid glycosides, dissolving the concentrate in water, and contacting the aqueous solution with a macro-porous resin, so that the phenylethanoid glycosides are adsorbed on the macro-porous resin; and
f) eluting the macro-porous resin with a fourth polar solvent and a fifth polar solvent in sequence, wherein the fifth polar solvent is lower in polarity than the fourth polar solvent, so that an eluate resulting from the fourth polar solvent elution does not contain acteoside and isoacteoside, and an eluate resulting from the fifth polar solvent elution contain only acteoside and isoacteoside, wherein the fourth polar solvent and the fifth polar solvent are a mixture of water and methanol or a mixture of water and ethanol.

11. The pharmaceutical composition for use as in claim 10, wherein the fourth polar solvent is 25-35% ethanol aqueous solution and the fifth polar solvent is 35-45% ethanol aqueous solution.

## Patentansprüche

1. Pharmazeutische Zusammensetzung zur Verwendung bei einem Verfahren zum Verhindern oder Behandeln von Amyloid-β-Peptid-assoziierten Erkrankungen oder Leiden bei einem Organismus, wobei die Zusammensetzung Acteosid und Isoacteosid als wirksame Bestandteile umfasst, wobei ein Gewichtsverhältnis des Isoacteosids zum Acteosid 4:1 bis 2:3 beträgt, wobei es sich bei der Amyloid-β-Peptid-assoziierten Erkrankung oder dem Amyloid-β-Peptid-assoziierten Leiden um die Alzheimer-Erkrankung, leichte kognitive Beeinträchtigung, Lewy-Körper-Demenz, das Down-Syndrom, den holländischen Typ der hereditären zerebralen Hämorrhagie mit Amyloidose (HCHWA), den Parkinson-Demenz-Komplex auf Guam, die zerebrale Amyloidangiopathie, Einschlusskörpermyositis, frontotemporale Demenz, altersbedingte Makuladegeneration oder Pick-Krankheit handelt.

2. Pharmazeutische Zusammensetzung zur Verwendung wie in Anspruch 1, welche frei von Echinacosid ist.

3. Pharmazeutische Zusammensetzung zur Verwendung wie in Anspruch 1, wobei die pharmazeutische Zusammensetzung die Bildung, Akkumulation oder Aggregation der Amyloid β-Peptide hemmt.

4. Pharmazeutische Zusammensetzung zur Verwendung wie in Anspruch 1, wobei die pharmazeutische Zusammensetzung zum Behandeln der Alzheimer-Erkrankung bestimmt ist.

5. Pharmazeutische Zusammensetzung zur Verwendung wie in Anspruch 1, wobei die pharmazeutische Zusammensetzung zum Verhindern, dass ein Organismus an der Alzheimer-Erkrankung leidet, oder zum Verzögern, dass ein Organismus an der Alzheimer-Erkrankung leidet, bestimmt ist.

6. Pharmazeutische Zusammensetzung zur Verwendung wie in Anspruch 1, wobei eine effektive Dosierung der pharmazeutischen Zusammensetzung an einen Menschen 0,2 mg - 4,0 mg der wirksamen Bestandteile pro kg Körpergewicht pro Tag entspricht.

7. Pharmazeutische Zusammensetzung zur Verwendung wie in Anspruch 2, welche eine von einer Pflanze als Quelle der wirksamen Bestandteile extrahierte Phenylethanoidglycosid-Herstellung umfasst, wobei die Herstellung das Isoacteosid und das Acteosid als die hauptsächlichen Phenylethanoidglycoside umfasst und der Gehalt an dem Isoacteosid höher ist als der an dem Acteosid.

8. Pharmazeutische Zusammensetzung zur Verwendung wie in Anspruch 7, wobei die Herstellung 12 - 32 % Acteosid und 26 - 46 % des Isoacteosids, auf Basis des Gewichts der Herstellung, umfasst.

9. Pharmazeutische Zusammensetzung zur Verwendung wie in Anspruch 8, wobei es sich bei der Pflanze um *Cistanche tubulosa* (Schenk.) Wight handelt.

10. Pharmazeutische Zusammensetzung zur Verwendung wie in Anspruch 9, wobei die Herstellung durch einen Vorgang bereitgestellt wird, umfassend die folgenden Schritte:
a) Extrahieren fleischiger Stängel von *Cistanche tubulosa* (Schenk.) Wight mit einem ersten polaren Lösungsmittel;
b) Einführen des aus Schritt a) resultierenden Extrakts in eine Säule, die mit hydrophoben makroporösen Polymerkügelchen gepackt ist, wodurch ermöglicht wird, dass Phenylethanoidglycoside auf die Polymerkügelchen adsorbiert werden können;
c) Eluieren der Säule unter Verwendung eines zweiten, als mobile Phase dienenden polaren Lösungsmittels, so dass relativ weniger stark adsorbierte Verbindungen von der Säule eluiert werden, während die meisten Phenylethanoidglycoside noch auf den Polymerkügelchen adsorbiert sind, und
d) Eluieren der Säule unter Verwendung eines dritten polaren Lösungsmittels, um so ein Eluat zu erhalten, welches Phenylethanoidglycoside enthält, wobei das erste polare Lösungsmittel Wasser, Methanol, Ethanol, ein Gemisch aus Wasser und Methanol oder ein Gemisch aus Wasser und Ethanol ist; das zweite polare Lösungsmittel Wasser ist und das dritte polare Lösungsmittel Methanol, Ethanol, ein Gemisch aus Wasser und Methanol oder ein Gemisch aus Wasser und Ethanol ist und das dritte polare Lösungsmittel eine geringere Polarität aufweist als das zweite polare Lösungsmittel;
e) Konzentrieren des Eluats, welches Phenylethanoidglycoside enthält, Lösen des Konzentrats in Wasser und In-Kontakt-Bringen der wässrigen Lösung mit einem makroporösen Harz, so dass die Phenylethanoidglycoside auf das makroporöse Harz adsorbiert werden, und
f) Eluieren des makroporösen Harzes mit einem vierten polaren Lösungsmittel und einem fünften polaren Lösungsmittel in Folge, wobei das fünfte polare Lösungsmittel eine geringere Polarität aufweist als das vierte polare Lösungsmittel, so dass ein aus der Elution mit dem vierten polaren Lösungsmittel resultierendes Eluat kein Acteosid und Isoacteosid enthält und ein aus der Elution mit dem fünften polaren Lösungsmittel resultierendes Eluat nur Acteosid und Isoacteosid enthält, wobei das vierte polare Lösungsmittel und das fünfte polare Lösungsmittel ein Gemisch aus Wasser und Methanol oder ein Gemisch aus Wasser und Ethanol sind.

11. Pharmazeutische Zusammensetzung zur Verwendung wie in Anspruch 10, wobei das vierte polare Lösungsmittel eine 25 - 35 %ige wässrige Ethanol-Lösung ist und das fünfte polare Lösungsmittel eine 35 - 45 %ige wässrige Ethanol-Lösung ist.

## Revendications

1. Composition pharmaceutique destinée à être utilisée dans un procédé de prévention ou de traitement de maladies ou d'affections associées au peptide β-amyloïde dans un organisme, ladite composition comprenant de l'actéoside et de l'isoactéoside comme principes actifs, un rapport massique de l'isoactéoside à l'actéoside allant de 4/1 à 2/3, la maladie ou l'affection associée au peptide β-amyloïde étant la maladie d'Alzheimer, une déficience cognitive légère, une démence à corps de Lewy, le syndrome de Down, une hémorragie cérébrale héréditaire avec amylose type néerlandais (HCHWA), le syndrome de Guam, une angiopathie amyloïde cérébrale, une myosite à inclusions, une démence fronto-temporale, une dégénération maculaire liée à l'âge ou la maladie de Pick.

2. Composition pharmaceutique destinée à être utilisée selon la revendication 1, qui est exempte d'échinacoside.

3. Composition pharmaceutique destinée à être utilisée selon la revendication 1, la composition pharmaceutique empêchant la formation, l'accumulation ou l'agrégation des peptides β-amyloïdes.

4. Composition pharmaceutique destinée à être utilisée selon la revendication 1, ladite composition pharmaceutique étant destinée à traiter la maladie d'Alzheimer.

5. Composition pharmaceutique destinée à être utilisée selon la revendication 1, ladite composition pharmaceutique étant destinée à éviter qu'un organisme souffre de la maladie d'Alzheimer, ou à retarder le fait qu'un organisme souffre de la maladie d'Alzheimer.

6. Composition pharmaceutique destinée à être utilisée selon la revendication 1, un dosage efficace de ladite composition pharmaceutique chez un humain étant équivalent à 0,2 mg à 4,0 mg desdits principes actifs par kg de poids corporel et par jour.

7. Composition pharmaceutique destinée à être utilisée selon la revendication 2, qui comprend une préparation de glycosides phényléthanoïdes extraite d'une plante en tant que source des principes actifs, la préparation comprenant l'isoactéoside et l'actéoside en tant que glycosides phényléthanoïdes principaux, et la teneur de l'isoactéoside étant supérieure à celle de l'actéoside.

8. Composition pharmaceutique destinée à être utilisée selon la revendication 7, la préparation comprenant 12 à 32 % d'actéoside et 26 à 46 % d'isoactéoside sur la base du poids de la préparation.

9. Composition pharmaceutique destinée à être utilisée selon la revendication 8, la plante étant *Cistanche tubulosa* (Schenk) Wight.

10. Composition pharmaceutique destinée à être utilisée selon la revendication 9, la préparation étant obtenue par un procédé comprenant les étapes suivantes :
a) extraction de tiges charnues de *Cistanche tubulosa* (Schenk) Wight avec un premier solvant polaire ;
b) introduction de l'extrait obtenu à l'étape a) dans une colonne garnie de billes polymères macroporeuses hydrophobes, ce qui permet aux glycosides phényléthanoïdes d'être adsorbés sur les billes polymères ;
c) élution de la colonne à l'aide d'un deuxième solvant polaire servant de phase mobile, de sorte que les composés adsorbés relativement moins fortement soient élués hors de la colonne, la plupart des glycosides phényléthanoïdes étant toujours adsorbés sur les billes polymères ; et
d) élution de la colonne à l'aide d'un troisième solvant polaire de façon à obtenir un éluat qui contient les glycosides phényléthanoïdes, où le premier solvant polaire est l'eau, le méthanol, l'éthanol, un mélange d'eau et de méthanol, ou un mélange d'eau et d'éthanol ; le deuxième solvant polaire est l'eau ; et le troisième solvant polaire est le méthanol, l'éthanol, un mélange d'eau et de méthanol, ou un mélange d'eau et d'éthanol, et le troisième solvant polaire est moins polaire que le deuxième solvant polaire ;
e) concentration de l'éluat qui contient les glycosides phényléthanoïdes, dissolution du concentré dans l'eau et mise en contact de la solution aqueuse avec une résine macroporeuse, de façon à adsorber les glycosides phényléthanoïdes sur la résine macroporeuse ;
f) élution de la résine avec un quatrième solvant polaire et un cinquième solvant polaire à la suite, le cinquième solvant polaire étant moins polaire que le quatrième solvant polaire, de sorte qu'un éluat résultant de l'élution avec le quatrième solvant polaire ne contienne pas d'actéoside et d'isoactéoside, et qu'un éluat résultant de l'élution avec le cinquième solvant polaire contienne uniquement de l'actéoside et de l'isoactéoside, le quatrième solvant polaire et le cinquième solvant polaires étant un mélange d'eau et de méthanol ou un mélange d'eau et d'éthanol.

11. Composition pharmaceutique destinée à être utilisée selon la revendication 10, le quatrième solvant polaire étant une solution aqueuse d'éthanol de 25 à 35 % et le cinquième solvant polaire étant une solution aqueuse d'éthanol de 35 à 45 %.
